# EUROPEAN PATENT APPLICATION

(11) **EP 4 257 680 A1**
(43) Date of publication of application: **11.10.2023**
(21) Application number: 22382336.0
(22) Date of filing: 07.04.2022
(51) Int. Cl.: C12N 9/02, C12P 5/02, C12P 7/18, C12P 7/26, C12P 7/44

(54) **METHOD FOR BIODEGRADING POLYOLEFIN-DERIVED POLYMERS**

(71) Applicant: Consejo Superior de Investigaciones Científicas (CSIC), 28006 Madrid (ES)
(72) Inventor: Bertocchini, Federica, Madrid (IT); Sola i Villarrubias, Maria, Madrid (ES)
(74) Representative: Pons

(57) **Abstract**

The invention refers to the use of an enzyme from the wax worm (*Galleria mellonella* larvae) saliva which has the unexpected capacity of oxidizing and depolymerizing untreated polyolefin-derived polymers, such as polyethylene (PE), at room temperature (RT), neutral pH and short incubation times. The invention also refers to a method for biodegrading a polyolefin-derived polymer wherein this enzyme is used.

## Description

The invention falls within the field of biotechnology, in particular within plastics, preferably polyolefin-derived plastics, more preferably polyethylene (PE), enzyme biodegradation.

### BACKGROUND ART

Polyethylene (PE) accounts for 30% of synthetic plastic production, largely contributing to plastic waste pollution on the planet to-date. Together with polypropylene (PP), polystyrene (PS) and polyvinylchloride (PVC), PE is one of the most resistant polymers, with very long C-C chains organized in a crystalline, dense structure. Given the hundreds of million tons of plastic waste accumulating and the still escalating pace of plastic production, re-utilization of plastic residues is a necessary path to alleviate the gravity of the plastic pollution problem, and at the same time to render available a huge potential reservoir of carbon. To-date, only mechanical recycling is being applied at a large scale. Several factors, such as the low number of plastic types prone to be mechanically recycled and the low quality of the secondary products, severely restrict the potential of this solution to the problem of plastic waste accumulation. Chemical recycling, as an alternative procedure, is preferentially aiming at plastic upcycling, e.g. decomposing polyolefin-derived plastics in order to take advantage of smaller intermediates. Several technologies have been applied at a lab scale, although the high energetic cost might still impede the scaling up of these technological tools.

In addition to mechanical and chemical recycling, biodegradation is widely considered as a promising strategy to dispose of plastic residues. Biodegradation refers to environmental degradation by biological agents. The IUPAC defines biodegradation as the "breakdown of a substance catalyzed by enzymes *in vitro* or *in vivo*", later modified "to exclude abiotic enzymatic processes". In the case of PE, biodegradation requires the introduction of oxygen into the polymeric chain; this causes the formation of carbonyl groups and the subsequent scission of the long hydrocarbon chains with production of smaller molecules, which can then be metabolized by microorganisms (Albertsson, A.C., Andersson, S. O. and Karlsson, S. (1987). Polymer Degradation and Stability 18, 73-87; Roy, P.K., Hakkarainen, M., Varma, I.K., and Albertsson, A.C. (2011). Environ Sci Technol 45, 4217-4227). The crucial first step of this chain of events, i.e. the oxidation of PE polymer, is usually carried out by abiotic factors such as light or temperature. Once the long polymeric molecules are broken down, a process that takes years of exposure to environmental factors in the wild, bacteria or fungi intervene and continue the job. This is the current paradigm driving the research field in biodegradation. Within this paradigm, several bacterial and fungal strains have been identified as capable of carrying on a certain extent of PE degradation. However, in most of the cases such degradation requires an aggressive pre-treatment of PE (heating, UV light, etc.) that accelerates the incorporation of oxygen into the polymer, making the abiotic oxidation the real bottleneck of the reaction (Wei, R., and Zimmermann, W. (2017). Microbial Biotechnology 10, 1308-1322; Restrepo-Florez, J.-M., Bassi, A. and Thompson, M. R. (2014). International Biodeterioration & Biodegradation 88, 83-90; Amobonye, A., Bhagwat, P., Singh, S., and Pillai, S. (2021). Sci Total Environ 759, 143536; Matjaŝiĉ, T., et al. (2021). Science of the Total Environment752*;* Walsh, A.N., et al. (2021). Environ. Sci. Technol 55, 12383-12392). In the past decade a few microorganisms have been described as capable of acting on untreated PE17-23, although they require a significantly longer incubation time compared to experimental conditions with pre-oxidized PE.

The identification of enzymes from microorganisms capable of degrading untreated PE has proven a difficult task. In fact, no such enzyme has been identified yet, confirming the crucial limiting role of oxidation in the whole biodegradation chain. Reported enzymes capable of acting on polyolefin-derived plastics require a pre-treatment of the plastic material (Wei, R., and Zimmermann, W. (2017). Microbial Biotechnology 10, 1308-1322; Amobonye, A., Bhagwat, P., Singh, S., and Pillai, S. (2021). Sci Total Environ 759, 143536.). For example, two reported laccases, able to chemically modify PE, necessitate an abiotic pre-treatment or the addition of redox mediators such as 1-hydroxybenzotriazole.

This scenario confirms that the synthetic nature of the compound, together with the hydrophobicity and inaccessibility features, make plastic a difficult target for animal, fungal or microbial-derived enzymatic activities. Nonetheless, some lepidopteran and coleopteran insects revealed the unexpected capacity to degrade untreated PE and PS, for example that described in Yang, Y., Wang, J., and Xia, M. (2020). Sci Total Environ 708, 135233.

However, it is still needed to identify enzymes from microorganisms which are capable of biodegrading untreated PE, in particular of oxidating untreated PE polymers, avoiding thus the use of abiotic factors, such as light or temperature, long incubation times or aggressive pre-treatments of PE.

### DESCRIPTION OF THE INVENTION

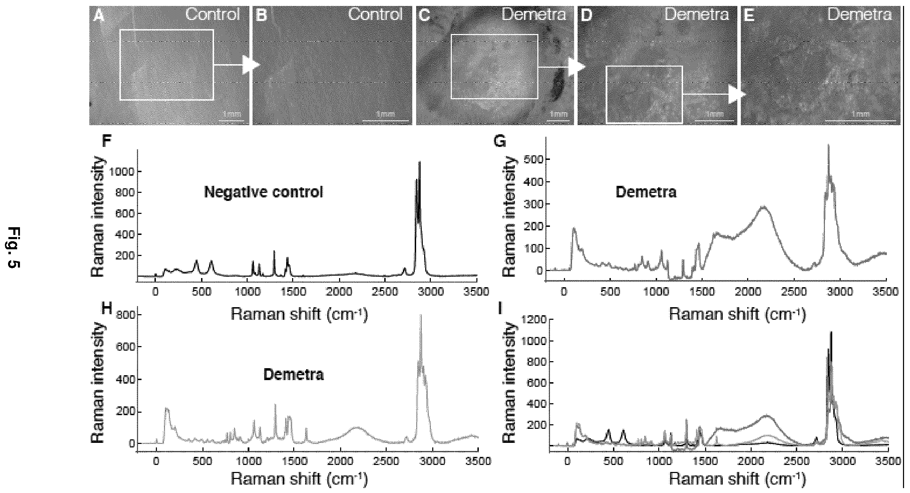

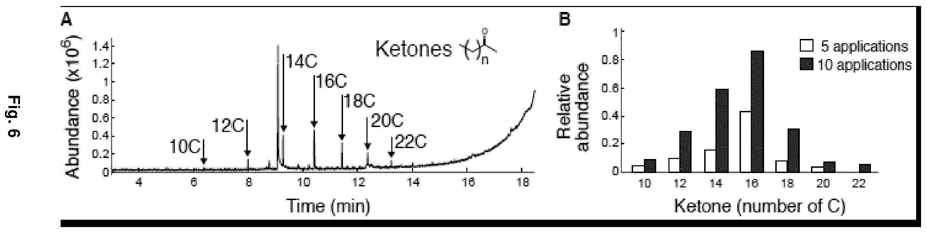

The inventors have isolated an enzyme from the wax worm (*Galleria mellonella* larvae) saliva which has the unexpected capacity of oxidizing and depolymerizing untreated polyolefin-derived polymers, such as polyethylene (PE), at room temperature (RT), neutral pH and short incubation times. They achieved this by carrying out a proteomic analysis and a size exclusion chromatography (SEC) of the wax worm saliva, obtaining one enzyme identified as arylphorin subunit alpha-like, re-named Demetra, with accession number XP_026756396.1 (NCBI), SEQ ID NO: 1 (the enzyme of the invention). The capacity of this enzyme to oxidize/degrade polyolefin-derived polymers was tested on PE films (see Example and figures 5 and 6 of the present patent application), showing a high degradation activity. This opens up a highway of possibilities to solve the plastic waste pollution issue.

This effect on PE degradation is achieved after only a few hours' exposure at room temperature and physiological conditions (neutral pH). Thus, no long incubations times or aggressive pre-treatments are needed. The enzyme provided in this invention can indeed overcome the bottleneck step in PE biodegradation, that is the initial oxidation step. Using Gas Chromatography-Mass Spectrometry (GC-MS) degradation products, such as small oxidized aliphatic chains, were identified, further confirming the breaking of the polymer in shorter molecules.

The enzyme of the invention belongs to the hexamerin/prophenoloxidase family. This enzyme is not only capable of oxidating PE after a few hours' application at room temperature, but also of physically deteriorating PE and releasing degradation by-products similar to the ones obtained with the whole saliva.

This enzyme is capable of producing such modifications on a PE film working at RT and in a very short time, embodying a promising alternative to the abiotic oxidation of plastic, the first and most difficult step in the degradation process. The identification of invertebrate enzymes capable of oxidizing PE in a few hours represents a totally new paradigm in the world of plastic degradation and more widely in the plastic waste management fields, and opens up a highway to design new formulae/routes for synthetic polymers production.

In view of the foregoing, in an aspect, the present invention relates to the use of:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1 (hereinafter, "the enzyme or the protein of the invention") or a functionally fragment thereof, or
- a host cell comprising a nucleotide sequence encoding said enzyme or a vector comprising a nucleotide sequence encoding said enzyme, (hereinafter, "the host cell of the invention"), or
- a composition comprising said enzyme or a functionally fragment thereof, or said host cell (hereinafter, "the composition of the invention"),
for biodegrading, or oxidating and/or depolymerizing, a polyolefin-derived polymer or a material comprising a polyolefin-derived polymer (hereinafter, "the use of the invention").

The enzyme of the invention is preferably isolated from the saliva of *Galleria mellonella* larvae, also known as wax worms (ww). It relates to the enzyme arylphorin subunit alpha-like, re-named Demetra, comprising an amino acid sequence having a sequence identity of, at least, 60% with sequence SEQ ID NO: 1 (accession reference number NCBI: XP_026756396.1).

SEQ ID NO: 1 (Demetra, the enzyme of the invention)

MFFNLWFHCNSVTVYFLTEYFILNNLFAVDPNLVNIQKKVLLLLENWKQV
DPDDEYYKIGKEYNIEANIESYTNREVVTEFLSLYKTGFTAKNQIFSIYY
ENQALEVRALYRLFYYAKDFETFYKTAAFARVWLNEGQFIYAFYIAVIHR

ADTRGIVLPAPYEIWPEYFVNSDVLAKINRIQMQKGLILPETAQYYGVLA
KDNAYYFYANYSGPWTYENNENLLSYFIEDVAWNSYYYYFHSKLQFWEKG
ENAIGPFKERRGEIYYFIYQQILARYYLERLSNGLGEIPRFNWNDRLQAG
YYPLLTTHQIPFAQRNGDYYLANDDNIEDIQFVDSYEKTFLQFLQKGQFK
AYKQEVDLYNSKSVNFVGNYWQANVDLYEKVPQRNYLRSYEDAARRILGA
APRNSYENLNVPTALDFYQTSLRDPAFYQLYAKILDFINQYKEYLEPYTQ
DVLHFVGVKINDVKVDKLVTYFEYFDWNATNAVYLSEQQLDTGSPSYIVR
QPRLNNQPFTVTIDIKSDVESEAVIKIFIGPKYDGNGYPIDLENNWVNLV
EIDWFTHKLTSGQNKIERKSENFFWFKEDSVSVSKIYELLNNGQVPRYMI
EKFLLLPRRLLLPRGTEGGVPFQFFVFVYPYQAPYKEWEPMKEFVVDNKP
FGYPFDRPVTESYYFTQPNMYFKDVYIYQEGEEYPYYTSYWSQNQVPKH

Alternatively, the enzyme of the invention may be recombinantly produced in accordance with techniques well-known in the art. For example, using accepted techniques of chemical synthesis, the enzyme may be built up either from the N-terminus or, more typically, the C-terminus using either single amino acids or peptides containing two or more amino acid residues. Particular techniques for synthesizing enzymes include classical methods, classical chemical synthesis amino acid by amino acid and solid phase peptide synthesis in which the enzyme is built up attached to a resin, such as a Merrifield resin. In these synthetic procedures, groups on the amino acids will generally be in a protected form using standard protecting groups such as t-butoxycarbonyl. If necessary, these protecting groups are cleaved once the synthesis is complete. Chemistry synthesis methods, for example by peptide synthesis in solid phase, solution synthesis, a combination of methods of solid phase synthesis and solution or enzymatic synthesis, are known by the experts in the field. The enzyme of the present invention may also be produced through recombinant DNA procedures known in the art. Modifications may be introduced during or after the synthesis of the enzyme, for example to include a label attached for purification, a purification tag.

In another particular embodiment, the enzyme of the invention is codified and expressed from a nucleotide sequence which is comprised in an expression vector.

In the present invention, the term "identity" or "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and TBLASTX, BLASTP and TBLASTN, are in the public domain at The National Center for Biotechonology Information (NCBI) website.

Persons skilled in the art understand that mutations in the nucleotide sequence of genes that lead to conservative amino acid substitutions at non-critical positions for the functionality of the protein are evolutionarily neutral mutations which do not affect its global structure or its functionality, giving rise to proteins which although comprise different amino acid sequence perform the same activity. These proteins are considered "functionally equivalent variants" of the sequence SEQ ID NO: 1 and fall within the scope of the present invention. Thus, the term "functionally equivalent variant", as used herein, means an enzyme which is derived from a native enzyme (SEQ ID NO: 1 in the present invention) by one or more deletions, insertions and/or substitutions of one or more amino acids at site(s) within its amino acid sequence and performs the same activity, i.e. it keeps the capacity of oxidizing untreated polyolefin-derived polymers, such as polyethylene (PE), at room temperature. Variants can be prepared using any mutagenesis procedure known in the art, such as site-directed mutagenesis, synthetic gene construction, semi-synthetic gene construction, random mutagenesis, shuffling, etc. All proteins having a sequence identity of at least 60% with the amino acid sequence of SEQ ID NO: 1 and capable of oxidizing untreated polyolefin-derived polymers are considered functionally equivalent variants in the context of the invention. An example of an assay to check if a given protein is a functionally equivalent variant of the enzyme of SEQ ID NO: 1 is disclosed in the examples of the present patent application. Functionally equivalent variants of the present invention have at least 20%, e.g., at least 40%, at least 50%, at least 60%, at least 70%, at least 80%, at least 90%, at least 95%, or at least 100% of the oxidizing activity of the native protein of SEQ ID NO: 1.

The present invention also encompasses functionally equivalent fragments of the enzyme of the invention. The term "functionally equivalent fragment" means a polypeptide/protein having one or more (e.g., several) amino acids absent from the amino and/or carboxy terminus of a native protein (in the present invention the sequence SEQ ID NO: 1) and shows the same activity/function that the native protein (in the present invention, the capacity of oxidizing untreated polyolefin-derived polymers at a room temperature). An example of an assay to check if a fragment of the protein of the invention is a functionally equivalent fragment of the SEQ ID NO: 1 is disclosed in the examples of the present patent application. Functionally equivalent fragments of the present invention have at least 20%, e.g., at least 40%, at least 50%, at least 60%, at least 70%, at least 80%, at least 90%, at least 95%, or at least 100% of the oxidizing activity of the native protein SEQ ID NO: 1.

In a preferred embodiment, the amino acid sequence of the enzyme of the invention comprises, or consists of, the sequence SEQ ID NO: 1.

The nucleotide sequence of the invention, encoding the enzyme of the invention, may further comprise other elements apart from the coding sequence, such as introns, non-coding sequences in the 3'and/or 5'ends, ribosome binding sites, or the like. The nucleotide sequence of the invention may also comprise sequences encoding for additional amino acids useful for increasing the enzyme stability or for allowing a more efficient enzyme purification.

The nucleotide sequence of the invention may be introduced into a vector or genetic construct, for example in a cloning or expression vector, in order to obtain a vector comprising said nucleotide sequence. Preferably, said vector is an appropriate vector for the expression and purification of the enzyme of the invention.

The term "genetic construct" or "vector", as used herein, refers to a nucleic acid molecule, monocatenary or bicatenary, that is isolated and modified to contain nucleic acid segments in a way that could not exist in nature. The term "nucleic acid construct" or "genetic construct" is synonymous to the term "expression cassette" when the nucleic acid construct contains the control sequences required for the expression of the nucleotide sequence of the invention. Therefore, the genetic construct of the invention may also comprise one or more control or regulatory sequences of gene expression, such as, but not limited to, promoter sequences, leader sequences, terminator sequences, polyadenylation sequences, signal sequences, regulators, enhancers, etc.

An "expression vector" is a linear or circular DNA molecule comprising at least the nucleotide sequence of the invention operationally linked to additional nucleotides provided for its expression. This vector that comprises the nucleic acid sequence of the invention can be introduced into a host cell in such a way that the vector is maintained as a chromosomal component or as an autoreplicating extracromosomal vector.

The expression vector referred to in the present invention may be any vector (e.g. plasmid or virus) that can be conveniently subjected to a recombinant DNA procedure and can produce the expression of the nucleotide sequence of the invention comprised in it. The choice of vector will normally depend on the compatibility of the vector with the host cell into which the vector is to be introduced. The expression vector can be, for example but not be limited to, a plasmid, a cosmid, a phage, a virus or viral vector, an artificial bacterial chromosome (BAC), an artificial yeast chromosome (YAC), or similar. Vectors within the context of the present invention can be linear or closed circular. Preferably, the expression vector of the invention is a baculovirus expression vector, more preferably a P2 baculovirus vector.

The "host cell", as used herein, includes any cell type which is susceptible to transformation, transfection, transduction, and the like with the nucleotide sequence or the expression vector of the invention as referred to above. The host cell may be prokaryote or eukaryote, preferably eukaryote, such as mammalian, insect, plant or fungal cell. In a preferred embodiment, the host cell is an insect cell, more preferably a sf9 cell.

The host cell of the invention comprises, therefore, at least the nucleic acid sequence of the invention, preferably by means of the vector of the invention, in a recombinant manner. The nucleotide of the invention or the vector of the invention is not naturally present in that cell, but has been intentionally introduced through genetic engineering procedures. The nucleotide sequence of the invention can encode the mature enzyme of the invention or a pre-protein consisting of a signal peptide linked to the mature enzyme that will have to be processed later in order to produce the mature enzyme.

The expression of the enzyme of the invention in the host cell of the invention may be induced by any procedure known in the art, such as the transformation of a suitable host cell with at least one nucleotide sequence of the invention or with the vector of the invention, and the culture of the transformed host cell under conditions that induce the expression of that nucleotide sequence in order to obtain the secreted and functional enzyme. Preferred conditions for inducing the expression of the nucleotide sequence are the incubation of the host cell at 27ºC for 48-72h.

The enzyme of the invention produced by the host cell host of the invention can be purified by a variety of procedures known in the art including, but not limited to, chromatography (e.g., ion exchange, affinity, hydrophobia, chromatofocus, and size exclusion), electrophoretic procedures (e.g. preparative isoelectric focusing), differential solubility (e.g. ammonium sulphate precipitation), SDS-PAGE, or extraction, in order to obtain a substantially pure enzyme.

In another preferred embodiment, the enzyme of the invention is recombinantly produced or it has been isolated from *Galleria mellonella,* particularly, from *G*. *mellonella* saliva.

Methods for isolating the enzyme of the invention from *Galleria mellonella* saliva are, without limitation chromatographic technics (such as size exclusion and ion exchange chromatgraphy) from wax worm saliva.

The composition of the invention comprises the enzyme of the invention or a functionally fragment thereof, or the host cell of the invention, and optionally other elements needed for the optimal activity of these or for their storage. These additional elements may be, for instance, buffers, other enzymes for example enzymes useful for biodegrading a polyolefin-derived polymer, antibiotics, or the like. Preferably, the composition of the invention further comprises a second enzyme comprising an amino acid sequence having a sequence identity of, at least, 60, 65, 70, 75, 80, 85, 90, 91, 92, 93, 94, 95, 96, 97, 98 or 99% with SEQ ID NO: 2, more preferably this additional protein comprises, or consist of, SEQ ID NO: 2.

SEQ ID NO: 2 (Ceres)

MGRLVLCVLALLVGGGISDPVKKLQRTVDQTVLDRQYKLLTLFFHPHEPI
HIKEQQEIAASWDLEKNIGLYENATAVHLTIQMLHNNYQVPRGVPFTVLE
SVHRFEISVYYSLLYSAKTYDTFYKTAVFLRQHVNENLFVNVLSVVILHR
SDTQDIRIPPIYDVFPSYFHNGEIMTTAQRITTHGQRMLEHYPSTYVWEN
NVVIRHNETAWPYYCNTESMPVSYFTHDVTLNALYYNIKLAYPIWLRSDA
CAIKEKRGELFFFWNKQLLARYYMERLSVGLGEIPELGLNEVEEGYVSGL
LYHNGIPYPVRPNHLVLNHQTWHAEAIEEIEVYENRIRDMIDQGFYITNT
GEHVSINSPDSIDVLGRLIEANVDSPNVQYYKDFISIWKKVLGNSLVHES
VAFNGIPLVVPSVLEQYQTALRDPAYYMIMKRVLKLFNLWHEHLPHYTTK
ELSVPSVKIEKVEVDKLLTYFEYTNFNVTNHLHLNEIECNNVINTKSVLV
QRTRLNHKVFTVRVNVKSGVAKHVTVRFFLAPKYDSVGNEIPLNVNTQNF
LLIDIFNYELKEGDNLITRVSSDNLLVTDEIDSASVLFNKVDSALQGHGQ
YMLNMKQNILKTPRHLLLPKGRVGGMPFVLMVYISEYHAPNDVHRGTVET
STIDNTIRLTSDTLGFPVDRPLFPWMLTGVENIFLQDVQIYHKPTTEVTGVPVYVE

In the present invention, "polyolefin-derived polymer" is a type of polymer with the general formula (CH₂CHR)ₙ where R is an alkyl group. They are usually derived from a small set of simple olefins (alkenes). Dominant in a commercial sense are polyethylene and polypropylene. More specialized polyolefins include polyisobutylene and polymethylpentene. They are all colorless or white oils or solids. The name of each polyolefin indicates the olefin from which it is prepared; for example, polyethylene is derived from ethylene, and polymethylpentene is derived from 4-methyl-1-pentene.

In another preferred embodiment, the polyolefin-derived polymer referred to in the present invention is polyethylene (PE).

"Polyethylene (PE)" or polythene (abbreviated PE; IUPAC name polyethene or poly(methylene)) is the most common plastic in use today. It is a polymer, primarily used for packaging (plastic bags, plastic films, geomembranes and containers including bottles, etc.). Many kinds of polyethylene are known, with most having the chemical formula (C₂H₄)ₙ. PE is usually a mixture of similar polymers of ethylene, with various values of n. It can be low-density or high-density: low-density polyethylene is extruded using high pressure (1000-5000 atm) and high temperature (520 kelvins), while high-density polyethylene is extruded using low pressure (6-7 atm) and low temperature (333-343 K). Polyethylene is usually thermoplastic, but it can be modified to become thermosetting instead, for example, in cross-linked polyethylene. All types of PE are encompassed within the scope of the present invention.

In an even more preferred embodiment, the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE). In a particular embodiment, the PE is LDPE, more preferably PE 4000 or PE 2000.

In another particular embodiment, the polyolefin-derived polymer or the material comprising a polyolefin-derived polymer is not pre-treated with abiotic factors (such as heating, UV light, etc,) previously to the biodegradation by the enzyme of the invention, the host cell of the invention or the composition of the invention, i.e. the enzyme of the invention is capable of biodegrading untreated polyolefin-derived polymer or untreated material comprising a polyolefin-derived polymer.

In another aspect, the present invention refers to a method, hereinafter "the method of the invention", for biodegrading, oxidating and/or depolymerizing a polyolefin-derived polymer, or a material comprising a polyolefin-derived polymer, wherein said method comprises contacting:
- the enzyme of the invention, or
- the host cell of the invention, or
- the composition of the invention,
with a polyolefin-derived polymer or a material comprising a polyolefin-derived polymer.

In a preferred embodiment of the method of the invention, said method is carried out at room temperature, preferably, at room temperature in an aqueous solution with a neutral pH.

"Room temperature" is from 15 °C to 30 °C, preferably 22°C.

"Neutral pH" is from pH 7 to pH 8.

In a preferred embodiment, the enzyme is used in the method of the invention in an amount between 2-10 µL, preferably 5 µL, and in a concentration between 1 and 5 µg/µL.

In another preferred embodiment of the method of the invention, the incubation time between the enzyme, the host cell or the composition of the invention, and the polyolefin-derived polymer is at least 60 to 120 min, preferably at least 90 min.

In an even more preferred embodiment of the method of the invention, the enzyme is used in an amount of 5 µL or 10 µL, in a concentration between 1 and 5 µg/µL, preferably 1.2 µg/µL, and is applied at least 8 times, preferably 24 times, on the polyolefin-derived polymer or a material comprising a polyolefin-derived polymer, 90 minutes each time.

In another preferred embodiment of the method of the invention, the enzyme of the invention comprises, or consists of, the sequence SEQ ID NO: 1. More preferably, the enzyme of the invention is isolated from *G. mellonella,* particularly, from *G. mellonella* saliva.

In another preferred embodiment of the method of the invention, the composition of the invention further comprises a second enzyme comprising an amino acid sequence having a sequence identity of, at least, 60, 65, 70, 75, 80, 85, 90, 91, 92, 93, 94, 95, 96, 97, 98 or 99% with SEQ ID NO: 2, more preferably comprising the SEQ ID NO: 2, even more preferably consisting of SEQ ID NO: 2.

In another preferred embodiment of the method of the invention, the polyolefin-derived polymer is polyethylene (PE).

In another preferred embodiment of the method of the invention, the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE). In a particular embodiment, the PE is LDPE, more preferably PE 4000 or PE 2000.

Another aspect of the invention refers to a method for obtaining by-products derived from the biodegradation of a polyolefin-derived polymer, comprising:
(a) contacting a polyolefin-derived polymer with
   - the enzyme of the invention, or
   - the host cell of the invention, or
   - the composition of the invention, and
(b) isolating the by-products obtained from the culture resulting from step (a).

"By-products", in the context of the present invention, are, but without limitation, butane, 2,3-Butanediol, trimethylslyl (TMS) derivative, sebacic acid, 2-ketones from 10 to 22 carbons, and a small aromatic compound recognizable as benzenepropanoic acid.

The conditions for this second method for obtaining by-products are those already explained above for the method for biodegrading, oxidating and/or depolymerizing a polyolefin-derived polymer, or a material comprising a polyolefin-derived polymer.

The isolation of the by-products in step (b) of this method may be performed by techniques well-known un the art, such as Gas Chromatography-Mass Spectroscopy (GC-MS).

### DESCRIPTION OF THE DRAWINGS

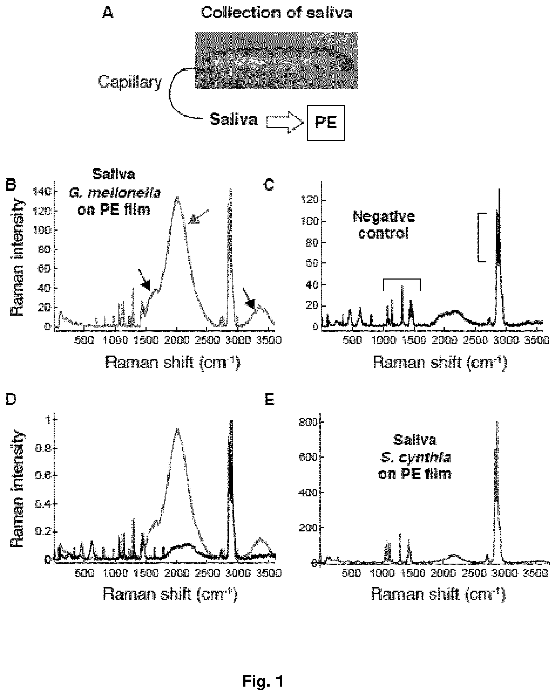

**Figure 1****. *Galleria mellonella* saliva (GmSal) collection and functional study.** A. Scheme of saliva collection and application. B, C, D, E. RAMAN analysis of PE film. B. PE film treated with GmSal: 3 applications of 90 minutes, 30 µl each. The peaks between 1500 and 2400 cm⁻¹ indicate different collective stretching vibrations due to the presence of other organic compounds, sign of PE deterioration (red arrow). Oxidation is indicated between 1600 and 1800 cm⁻¹ (carbonyl group) and 3000-3500 cm⁻¹ (hydroxyl group) (black arrows) (Larkin, 1998). C. Control PE film. Brackets indicate the picks that characterize PE (PE signature), corresponding to the bands at 1061, 1128, 1294, 1440, 2846 and 2880 cm⁻¹. D. Overlapping profiles (B and C). E. PE film treated with *Samia cynthia* saliva.

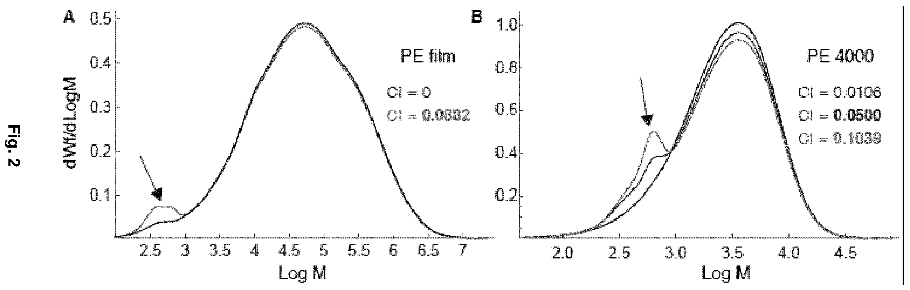

**Figure 2****. HT-GPC analyses of PE treated with GmSal.** Molecular weight distribution of PE film (A) and PE 4000 (B) are indicated. A. Control PE (in black) and PE-treated (in red) are compared. The carbonyl index (CI) of the control and experimental are indicated. M_{w} control (red): 0; M_{w} experimental (black): 0.0882. B. Control PE (black), treated-PE (15 applications) (blue), treated-PE (30 applications) are compared. The CI of each sample is indicated. M_{w} control (black): 0.0106; Mw PE treated-15 applications (blue): 0.0500; Mw PE treated (30 applications) (red): 0.1039. Arrows indicate compounds with low molecular weight in the treated PE.

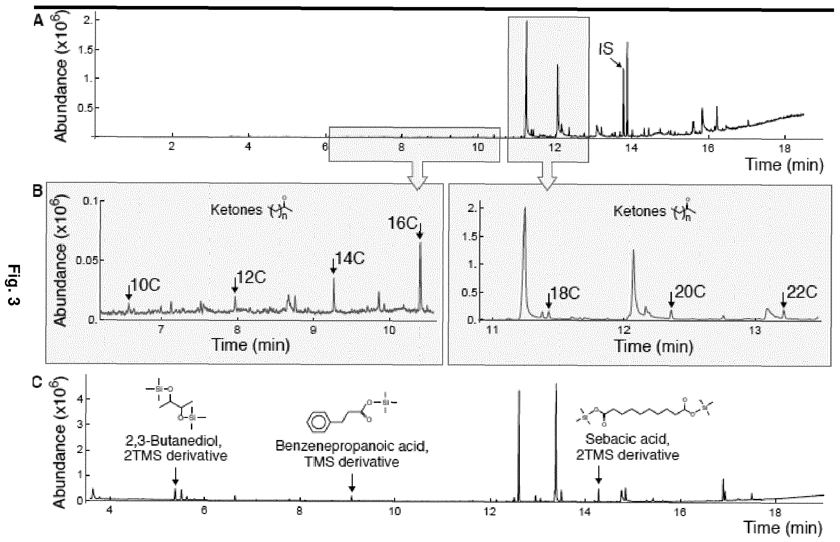

**Figure 3****. Identification of PE degradation by-products via GC-MS.** A, B, C. Chromatograms of PE treated with GmSal, indicating different compounds. A and B. Ketones of different length, indicated by the number of carbon atoms. C. 2,3-Butanediol 2TMS derivative, benzenepropanoic acid TMS derivative, sebacic acid sTMS derivative. Compounds in C were identified with silylation (see Methods). IS: internal standard.

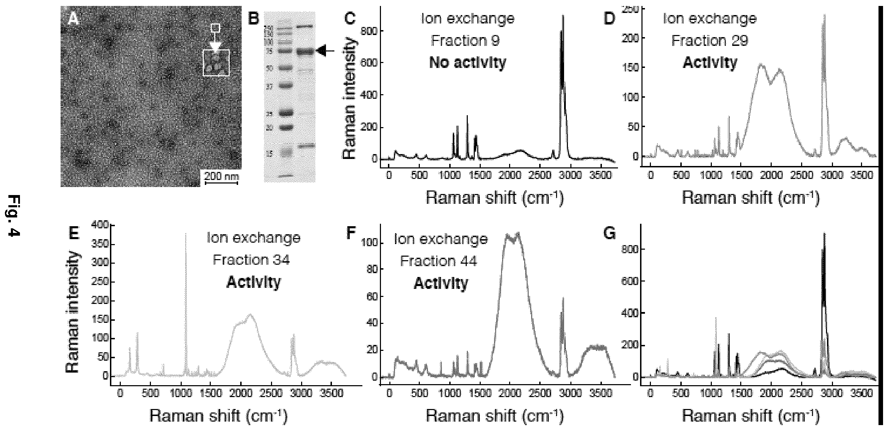

**Figure 4****. GmSal content functional characterization.** A. Electron microscopy negative staining of a saliva sample, dilution 1:500. Protein complexes (10-15 nm size) are indicated in the top right square. B. SDS-PAGE of a GmSal sample, dilution 1:50. Molecular weight standards are listed on the left. The arrow indicates the major band at 75kDa. C-F. RAMAN of PE treated with IEX fractions (see Figure S4). C. Fraction 9, no activity (control PE film, see Figure 1 for details). D-F. Profile corresponding to IEX peak 1 (fraction 29), 2 (fraction 34) and 3 (fraction 44) (see Fig 1 for details). The intense peak at 1085 cm⁻¹ in E indicates amorphous PE. The decrease of the peaks at 2845 and 2878 cm⁻¹ indicates a less significant presence of PE. G. Overlapping of fractions C to F.
**Figure 5****. Demetra effect on PE film.** A, B. Control PE film. C-E. Demetra on PE film. F-I. RAMAN spectroscopy of control (F) and Demetra treated PE film (G, H). F. Control PE film (see Figure 1 for details). G, H. Two different punctual analyses within the crater as showed in E, indicating PE deterioration. See Fig. 1 for details. Moreover, the bands at 845 and 916 cm⁻¹ correspond to C-O-C and C-COO groups, respectively. The presence of PE is insignificant in G.
**Figure 6****. Generation of PE degradation by-products by Demetra.** A. GC-MS chromatogram of PE treated with Demetra. The arrows indicated the peaks corresponding to ketones with different number of carbons. B. Increase of ketones formation as degradation products from five to ten applications of Demetra to PE.

### EXAMPLE

### I-MATERAIL AND METHODS

### Wax worm saliva collection

*Galleria mellonella* larvae of 150-300 mg were used for saliva collection (GmSal). Briefly, a glass capillary connected to a mouth pipet was placed at the buccal opening and the liquid was collected. Saliva for PE application was immediately used.

Occasionally, frozen saliva-only can be utilized. For electron microscopy, saliva was diluted 1:1 in the following buffer: 10mM Tris-CI pH 8, 50 mM NaCl. For proteomic analyses, saliva was diluted 1:1 in 10 mM Tris-CI, 50 mM NaCl, 2 mM DTT, 20% glycerol. For control, *Samia cynthia* larvae (kindly provided by InsectPark-Microfauna S.L. (Escorial, Madrid)) at the last stage were used to collect saliva as previously described.

### RAMAN and FTIR analyses

PE film was treated with 30 µl of GmSal for 90 minutes, three applications for RAMAN. PE film was treated with 5 µl of GmSal for 90 minutes, nine applications for FTIR. Recombinant proteins were applied as follows: 5 µl of protein (concentration between 1 and 5 µg/ml) were applied eight times on PE film 90 minutes each time. For the control with inactivated proteins, recombinant proteins were denatured at 100 degrees for 10 minutes. Size exclusion chromatography (SEC) and ion exchange chromatography (IEX) peak aliquots were applied six times, 30 minutes each, and left overnight. Treated and control films were washed with water and ethanol. RAMAN analyses were performed on (treated and control) PE films using Alpha300R - Alpha300AAFM Witec equipment with 5mW power, 50x (NA0.8) objective, integration time 1, accumulation 30, wavelength 532nm. FTIR analyses were performed with a Jasco LE-4200 equipment, with the following features: interval 4000-400 cm⁻¹, Resolution 4 cm⁻¹, scan 264.

### High Temperature-Gel Permeation Chromatography (HT-GPC) analysis.

HT-GPS was performed by Polymer Chart, Valencia, Spain. Briefly, the followings are the experimental conditions: Equipment: GPC-IR5_I Polymer Char; solvent: TCB stabilized with 300ppm of BHT; dissolution temperature, detectors temperature, columns Temperature: 160 degrees; volume: 8ml; weight: 8mg; dissolution Time: 60 minutes; injected volume: 200 µl; injection time: 55 minutes; flow: 1ml/minute; columns: 3 µ PL gel Olexis Mix-Bed columns (13 microns), 300 x 7.5mm + guard column.

For the carbonyl index analysis, GPC_IR6 was used, with dissolvent o-DCB and temperature at 150 degrees. PE film and PE 4000 were treated with 100 µl of GmSal for 90 minutes. The treatment was repeated 15 times (film and PE 4000) and 30 times (PE 4000).

### Gas Chromatography Mass Spectroscopy (GC-MS) and Tandem analysis.

An amount of 20 mg of PE 4,000 or 1.5 mg PE 2,000 were placed in a 1.5 mL Eppendorf tube. PE was exposed to 40 µL of *G. mellonella* saliva 9 times for 90 minutes each at room temperature and avoiding light. For prolonged treatment (day 1, 2, 3, and 6), three applications of 100 µL of saliva for 90 minutes each at room temperature were carried out each day. Controls of each PE were performed using Milli-Q water in substitution of the saliva of *G. mellonella* larvae, as well as saliva of *G. mellonella* larvae only. Also, PE was exposed to 10 µL (1.2 mg/mL) of Demetra (SEQ ID NO: 1) 24 times for 90 minutes. Prolonged treatment was performed as well for Demetra (days 1 and 2), five applications per day of 10 µL (1.2 mg/mL) for 90 minutes each. As control, the same experiment was repeated using the protein buffer. Afterward, samples were centrifuged with an Eppendorf centrifuge 5810 R at 19,083 G-force for 30 seconds and the subnatant was transferred to a new 1.5 mL Eppendorf tube. Samples and controls were extracted using a QuEChERS (quick, easy, cheap, effective, and safe) method1 based on2 with some modifications. Briefly, 50 µL of diphenyl phthalate (Internal Standard; IS) at a concentration of 1 mg/mL was added at each sample and extracted with 300 µL of dichloromethane (DCM) and 5 % (v/m) of NaCl. The tube was vortexed for 30 seconds and sonicated in a bath (50/60 Hz) for 15 minutes at room temperature, followed by centrifugation with an Eppendorf centrifuge 5810 R at 20 ºC and 19,083 G-force for 10 minutes. Finally, DCM located as the subnatant was collected and placed in an insert before analysis. Silylation reaction with N, O-Bis(trimethylsilyl)trifluoroacetamide (BSTFA) was performed to determine the low-volatility polar compounds which show low detection sensibility. A fraction of 50 µL of each sample with 50 µL of BSTFA was incubated for 20 minutes at 60 ºC before the analysis.

Dichloromethane (DCM; CAS-No: 75-09-2) for gas chromatography-mass spectrometry (GC-MS) was SupraSolv grade purity and obtained from Sigma-Aldrich (Darmstadt, Germany). Sodium chloride (NaCl; ≥ 99.5 %; CAS-No: 7647-14-5) and ultrapure water from a Milli-Q system were supplied from Merck (Darmstadt, Germany).

Crystalline granular powder polyethylene (PE 4,000; CAS-No: 9002-88-4) and analytical standard polyethylene (PE 2,000; CAS-No: 9002-88-4, details: Mw (Da) 1970; Mn (Da) 1700; Mp (Da) 1890; PD (Mw/Mn) 1.16) were supplied by Sigma-Aldrich (Saint Louis, USA).

Chromatographic analyses were performed with a gas chromatography-mass spectrometry system (GC-MS) 7980A-5975C from Agilent Technologies. Separation of the metabolites was performed on a DB-5th Column coated with polyimide (30 m length, 0.25 mm inner diameter, and 0.1 µm film thickness; Agilent Technologies, USA) for proper separation of substances, and Helium (He) was utilized as a carrier gas. The analysis was performed using a split injector at 350 ºC and an injection volume of 1 µL. The ion source temperature was 230 ºC, the ºC mass spectral analysis was performed in scan mode, the quadrupole temperature of 150 ºC, and a fragmentation voltage of 70 eV. The oven program started at 60 ºC for 3 minutes, then 20 ºC/minute to 350 ºC for 1 minute. The total run time was 18.5 minutes and 19.5 minutes for derivatized samples. The resulting chromatograms were processed using the software MSD ChemStation E.01.00.237 from Agilent Technologies, Inc while for the identification NIST11 library was used.

The evaluation of the prolonged treatment was based on the relative abundance of each untargeted compound, which consists of the quotient of the area under the peak of each compound divided by the area under the peak of the IS.

Gas Chromatography/Tandem Mass Spectrometry was used for confirmation of the non-target compounds by a BRUKER 456-GC SCION TQ. This experiment was performed by the Elemental and Molecular Analysis facility, University of Extremadura, Spain. Briefly, the injector port was set at 230 °C in Split mode. Separation was achieved using a column HP 5MS, 30 m, 0.25 mm, and 0.25 µm. Helium (He) was utilized as a carrier gas. The column oven was programmed in the following conditions: 60 °C for 3 minutes, increase of 20 °C/minute to 325 °C for 1 minute. The collision energy was 15 eV.

### Electron Microscopy analysis

Larvae saliva samples were diluted 1:50 in the proper buffer (see "Wax worm saliva collection")

Samples were analyzed by electron microscopy (EM) after being adsorbed to glow-discharged carbon coated grids and stained with 2% uranyl acetate. Grids were observed using a JEOL JEM-1230 EM operated at 100 kV and a nominal magnification of 40 000. EM images were taken under low dose conditions with a CMOS Tvips TemCam-F416 camera, at 2.84 Å per pixel.

### Protein Chromatography analyses

For the size exclusion chromatography, wax worms (ww) saliva in the proper buffer (see "Wax worm saliva collection") was thawed, pooled and centrifuged. The supernatant was filtered (0.45 µm cutoff, Ultrafree Millipore) and loaded to a size exclusion chromatography column Superdex 200 5-150 (Cytiva) equilibrated with 10 mM Tris-CI, 50 mM NaCl, 2 mM DTT.

For the ion exchange chromatography, upon thawing, the sample was diluted to 100 µL with 10 mM Tris-CI at pH 8, centrifuged, filtered and the supernatant loaded to a monoQ 5/50 GL ion exchange column (Cytiva). After a wash step, a 40mL gradient with buffer A (10 mM Tris-CI pH8), and buffer B (same as A supplemented with 500 mM NaCI) was applied.

### Proteomic analysis

Liquid Chromatography Mass Spectrometry (LC-MS) analysis. All peptide separations were carried out on an Easy-nLC 1000 nano system (Thermo Fisher Scientific). For each analysis, the sample was loaded into a precolumn Acclaim PepMap 100 (Thermo Fisher Scientific) and eluted in a RSLC PepMap C18, 15 cm long, 50 µm inner diameter and 2 µm particle size (Thermo Fisher Scientific). The mobile phase flow rate was 300 nl/minute using 0.1% formic acid in water (solvent A) and 0.1% formic acid and 100% acetonitrile (solvent B). The gradient profile was set as follows: 5%-35% solvent B for 45 min, 35%-100% solvent B for 5 minutes, 100% solvent B for 10 minutes. Four µl of each sample were injected.

MS analysis was performed using a Q Exactive mass spectrometer (Thermo Fisher Scientific). For ionization, 1900 V of liquid junction voltage and 270 °C capillary temperature was used. The full scan method employed a m/z 400-1500 mass selection, an Orbitrap resolution of 70,000 (at m/z 200), a target automatic gain control (AGC) value of 3e6, and maximum injection times of 100 ms. After the survey scan, the 15 most intense precursor ions were selected for MS/MS fragmentation. Fragmentation was performed with a normalized collision energy of 27 eV and MS/MS scans were acquired with a starting mass of m/z 100, AGC target was 2e5, resolution of 17,500 (at m/z 200), intensity threshold of 8e4, isolation window of 2 m/z units and maximum IT was 100 ms. Charge state screening was enabled to reject unassigned, singly charged, and equal or more than seven protonated ions. A dynamic exclusion time of 20s was used to discriminate against previously selected ions.

MS data analysis. Mass spectra *.raw files were searched against an in -house specific database against Galleria_Proteins (12715 proteins entries), using the Sequest search engine through Proteome Discoverer (version 1.4.1.14) (Thermo Scientific). Search parameters included a maximum of two missed cleavages allowed, carbamidomethyl of cysteines as a fixed modification and oxidation of methionine as variable modifications. Precursor and fragment mass tolerance were set to 10 ppm and 0.02 Da, respectively. Identified peptides were validated using Percolator algorithm with a q-value threshold of 0.01. The protein identification by nLC-MS/MS was carried out in the Proteomics and Genomics Facility (CIB-CSIC), a member of ProteoRed-ISCIII network3.

### Recombinant protein production and utilization

Arylphorin, Arylphorin subunit alpha-like (Demetra, SEQ ID NO: 1) and hexamerin (Ceres, SEQ ID NO: 2) were produced by Genscript, utilizing the baculovirus expression system in insect cells, according to the manufacturer. Briefly, sf9 cells were infected with P2 baculovirus, flasks were incubated at 27°C for 48-72 hours and media harvested. Then cells were removed, and transfection medium was applied for purification. The produced proteins were resuspended in 150 mM NaCl, 20 mM Hepes, 5% glycerol and used for the degradation assay. The same buffer alone was used as negative control.

### Galleria mellonella genome annotation

In order to obtain the most useful information from mass spectrometry analysis of proteins extracted from ww saliva, it is pivotal to use a representative database of protein sequences. In addition to the NCBI official annotation of *G. mellonella* protein sequences, a new annotation was produced exploiting also the information of *G*. *mellonella* salivary glands RNA-seq data (Bertocchini, unpublished). *G mellonella* genome annotation was performed using the genome sequence available at NCBI (accession number ASM258982v1). A specific pipeline was developed to combine the information from RNA-seq (Bertocchini, unpublished) with ab initio predictors in order to obtain the most accurate annotation. Briefly, the RNA-seq data was mapped on the reference genome using STAR (version 2.5 0c) in local mode and used to perform a reference guided transcriptome assembly with Trinity (v2.11.0). The obtained transcripts and the mapping files were used as input for the Braker2 pipeline to combine AUGUSTUS ab initio annotation with the transcriptome assembly to obtain the annotation in GFF format, together with transcript and protein sequences. Proteins were used as input for the PANNZER2 pipeline to obtain descriptions8, Gene Ontology and KEGG annotations. About 32000 genes could be annotated in the Galleria genome. The corresponding proteins were analyzed to assess their completeness performing a BLASTP alignment against the UniRef90 database and calculating the percentage of alignment. A similarity search against the UniRef90 (Nov2018) database showed that about 50% of the predicted proteins covered 100% of the corresponding hits (i.e. full length) and that about 80% of the predicted proteins covered at least 50% of the corresponding hits. As a further control, the proteins and the genome were evaluated with the BUSCOv2 pipeline. The BUSCO database contains sets of single-copy highly conserved genes across different taxa (i.e. Eukaryota or Insects). By performing an analysis with the BUSCO database it is possible to assess the completeness of a genome/proteome, the presence of duplications and/or fragmentations. This analysis was performed using the predicted proteome and also the unannotated genome for a comparison. By comparing the results of the unannotated with the annotated genome, we can see a small fraction of missing genes which are probably absent from the genome assembly and that cannot be recovered from the current genome sequence. This explains some missing genes present in the later NCBI annotation.

### II - RESULTS

### Wax worm saliva oxidizes PE film

Saliva, broadly defined here as the juice present in the anterior portion of the digestive apparatus, was collected from the ww mouth and tested on a commercial PE film (Fig. 1A). After three consecutive applications of 30 µl of GmSal for 90 minutes each, Confocal Raman microscopy/Raman spectroscopy (RAMAN) analysis indicated a highly oxidized polymer, accompanied by a general deterioration of the film (Fig. 1B). This is evident in the overlapping with the PE control (Fig. 1C, D), which reveals the expected PE signature profile. As a further control, the saliva of another lepidopteran larva, *Samia cynthia,* was applied on the PE film, and no oxidation was generated (Fig. 1E). The changes produced by the GmSal in a few hours-long applications are similar to those generated by environmental factors after months or years of exposure to weathering. The Fourier Transformed Infrared Spectroscopy (FTIR) analysis confirmed the oxidation profile. The changes in PE chemical composition revealed by the spectroscopy techniques suggested that molecules other than the long PE polymeric chain formed upon the contact with GmSal.

### Wax worm saliva degrades PE

The molecular weight characteristics of PE before and after treatment with GmSal were analysed using High Temperature-Gel Permeation Chromatography (HT-GPC). After a few hours' applications of GmSal on PE film (15 applications, 90 minutes each), the molecular weight distribution became bi-modal, with a new peak in the low molecular weight region, indicating the breaking of the C-C bonds with the appearance of small compounds (Fig. 2A). Formation of C=O bonds as a consequence of chain scission was shown by the increase of the carbonyl index (CI, Fig. 2A). The weight average (M_{w}) was only slightly changed suggesting that the polymer was not uniformly modified by the GmSal, with portions that have been strongly depolymerized, while others remained still untouched. The same result was obtained using PE 4000 instead of PE film with an increase in the carbonyl index in the GmSal-treated sample (Fig. 2B, black and blue curves). These results show that PE was oxidized and depolymerized as a consequence of GmSal exposure, with formation of oxidized molecules of low molecular weight. In order to analyse the dynamics of changes in the PE after increased exposure to GmSal in time, the exposure time of PE 4000 to GmSal was doubled (30 applications, 90 minutes each) (Fig. 2B, red curve). Breaking of large polymer chains with formation of smaller molecules notably increased at longer exposure times, as showed by the comparison of the molecular weight distributions and by the doubling of the CI (Fig. 2B). These experiments confirmed the PE oxidation with depolymerization upon a few hours' exposure to GmSal, an effect that increased with time, and pointed to the presence therein of still unknown activities capable of PE degradation.

### Identification and analysis of the by-products of PE treated with wax worm saliva

To have an insight into the degradation products resulting from PE-saliva contact and released from the oxidized polymer, PE granules (crystal polyethylene-PE 4000) were exposed to GmSal and subsequently analysed by Gas Chromatography-Mass Spectrometry (GC-MS) and identified by NIST11 library for untargeted compounds. After 9 applications of 40 µL of GmSal for 90 minutes each at RT, new compounds were detected in the experimental sample (Fig. 3). The detected compounds comprised oxidized aliphatic chains, like 2-ketones from 10 to 22 carbons. Ketones from 10 to 18 carbons were identified by comparing the fragmentgram of the ion m/z 58 from methyl ketones that correspond with the transposition of the McLafferty on the carbonyl located at the second carbon of each ketone. Those not present in the library as 2-eicosanone and 2-docosanone showed the same fragmentgram m/z 58 and were defined by the equidistance of the peaks along the retention time and their molecular weight. Furthermore, the presence of 2-ketones was confirmed with GC-MS/MS in MRM mode with the ion with the highest m/z and the exclusive of each molecule as 212>58 for 2-tetradecanone, 240>59 for 2-hexadecanone, and 282>58 for 2-octadecanone. Also, butane, 2,3-Butanediol, 2-trimethylslyl (TMS) derivative, and sebacic acid, 2TMS derivative were identified using sample silylation, indicating the deterioration of the PE chain (Fig. 3C). At the same time, a small aromatic compound recognizable as benzenepropanoic acid, TMS derivative, a plastic antioxidant, was found. Derivative chemicals were confirmed as well using GC-MS/MS with an m/z of 147>73, 331 >73, and 104>75, respectively. With the exception of ketones, the same results were obtained using monodispersed polyethylene-PE 2000. The presence of this plastic antioxidant suggests an "opening" in the polymeric structures, with the release of small stabilizing compounds normally present in plastics (plastic additives).

To verify if an increase in time exposure to GmSal caused an increase in PE degradation, it was repeated the experiment of PE 4000 exposure in sequential times, with four applications per day of 100 µL of GmSal for 90 minutes each at RT, performed in 1, 2, 3 and 6 consecutive days. The analysis of the supernatants revealed a progressive increase in the formation of 2-decanone, 2-dodecanone, 2-tetradecanone, and 2-hexadecanone. These data indicate an increase of at least twice the relative abundance in the degree of polymer oxidation and degradation with time (1 to 6 days) as a consequence of prolonged exposure to the GmSal.

### Study of the wax worn saliva: enzymes identification and functional studies

To understand the nature of the buccal juice, a GmSal sample was analysed by negative staining electron microscopy (EM), revealing a high content of proteins or protein complexes (size:10-15 nm) (Fig. 4A). No other structures (such as vesicles or bacteria) were detected. Electrophoretic analysis (SDS-PAGE) confirmed the presence of proteins with a prominent band at around 75kDa (Fig. 4B).

To assess if GmSal contains enzymes responsible for the detected PE modifications, a proteomic analysis of GmSal contents was carried out. More than 200 proteins were detected, including a variety of enzymatic activities, transport and structural proteins, etc. To narrow down the number of potential candidates, a saliva sample was analyzed by size exclusion chromatography (SEC). The elution profile showed a main single, wide peak. SDS-PAGE gel of the major fraction showed a strong band at about 75kDa. The proteomic profile of this band revealed the presence of proteins known in arthropods as related to transport or storage.

To further identify if the wide peak contained subspecies, an ion exchange chromatography (IEX) was run with a second saliva sample, which showed four well-defined elution peaks. Analysis by SDS-PAGE indicated that they all contained proteins of similar molecular weight. To check which protein fractions of both the SEC and IEX retained PE degradation activity, aliquots of the eluted fractions were tested on a PE film. Using RAMAN spectroscopy, degradation activity was analysed from fractions of the IEX four peaks (Fig. 4C-G) and in the SEC major peak. Peaks indicated as 1, 2 and 3 showed a major extent of PE oxidation than fraction 4, which evidently contained some extent of residual activity (not shown). High degradation activity was also detected in the SEC main peak.

Proteomics of IEX peaks 1, 2, and 3 revealed the presence of a handful of proteins, belonging to the arthropodan hexamerin/prophenoloxidase superfamily. This result on the one hand confirmed the outcome of the SEC fraction proteomics, and on the other refined it, reducing the number of potential candidates present in each peak. The fact that this family comprehends oxidase activities, made them the candidates for PE degradation capacity within GmSal. These proteins, namely arylphorin subunit alpha, arylphorin subunit alpha-like, and the hexamerin acidic juvenile hormone-suppressible protein 1 were produced using a recombinant expression system and tested for this ability.

### Identification of wax worm enzymes as PE oxidizers

In order to assess the activity of these proteins, 5 µL of each purified enzyme at a concentration of 1-5 µg/µL, were applied separately 8 sequential times (90 minutes each) on PE films. While arylphorin subunit alpha did not show any effect on PE film (not shown), arylphorin subunit alpha-like, re-named Demetra (accession number: XP_026756396.1 SEQ ID NO: 1), caused PE deterioration with occasional conspicuous visual effect on the film itself (Fig. 5A-E). RAMAN confirmed oxidation and a damaged PE signature (Fig. 5F-H). The hexamerin, re-named Ceres (accession number: XP_026756459.1 SEQ ID NO: 2), caused PE oxidation/deterioration, but without the visual effect caused by Demetra. The same experiment performed with inactivated enzymes did not show any modification on the PE film, as indicated by RAMAN analysis. These results support the original hypothesis based on the idea that the buccal secretion is the main source of plastic degradation activities in the ww.

To analyse the potentiality of the saliva proteins in oxidizing PE, GC-MS was performed on PE granules (PE 4000) exposed to Demetra (SEQ ID NO: 1) or Ceres (SEQ ID NO: 2). After 24 applications of Demetra (10 µL at 1.2 µg/µL, 90 minutes each), 2-ketones from 10 to 22 carbons were detected in the supernatant using GC-MS, the fragmentgram m/z 58, and retention time for identifications (Fig. 6A).

Increasing the treatment (ten versus five applications, 90 minutes each) showed an increase of 2-ketones of 10 to 20 carbons in relative abundance, and the appearance of 2-docosanone which was not detected after five applications (Fig. 6B). On the other hand, after PE 4000 treatment with Ceres, no by products were detected in the supernatant, suggesting substantial difference between the two proteins, despite they both shared the capacity to oxidize PE.

This invention evidences that the saliva of the ww oxidizes and depolymerizes PE, with ww enzymes therein capable of reproducing the effect observed with the whole saliva. This is the first report of an enzymatic activity capable of attacking the PE polymer without any previous abiotic treatment. This capacity is achieved by animal enzymes working at room temperature and in aqueous solution with a neutral pH. Under these conditions, the enzymatic action of the ww saliva overcomes in a few hours a recognized bottleneck step (i.e. oxidation) in PE degradation.

The action on PE of the enzymes disclosed in this invention and present in the saliva of *G. mellonella* is equivalent to that of abiotic pretreatments.

The capacity of these particular enzymes (SEQ ID NO: 1 and SEQ ID NO: 2) to rapidly and extensively oxidize PE, a polymeric, compact hydrophobic substance, is unexpected. The existence of enzymes produced by insects, secreted from the mouth and evolved to work at room temperature and neutral pH on plastic provides a new paradigm for biological degradation of PE. This new framework goes well beyond the current definition of biodegradation, which is exclusively based on the full conversion of plastic to CO₂ through the metabolic activity of microorganisms: on one hand, the observed oxidation and deterioration of PE do not depend on any microbial activity; on the other hand, the easy working conditions and the appearance of degradation products such as ketones and additives suggest the use of these enzymes for plastic waste degradation and recycling or upcycling of plastic components. This potentiality could be used either as an alternative to the metabolic conversion of plastic to CO₂, or as the initial oxidative step in combination with standard microbial degradation pathways.

## Claims

1. Use of:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1, or
- a host cell comprising a nucleotide sequence encoding said enzyme, or
- a composition comprising said enzyme or said host cell,
for biodegrading a polyolefin-derived polymer or a material comprising a polyolefin-derived polymer.

2. Use according to claim 1, wherein the amino acid sequence comprises, or consists of, the sequence SEQ ID NO: 1.

3. Use according to claims 1 or 2, wherein the composition further comprises a second enzyme which comprises an amino acid sequence having a sequence identity of, at least, 60, 65, 70, 75, 80, 85, 90, 91, 92, 93, 94, 95, 96, 97, 98 or 99% with SEQ ID NO:2.

4. Use according to any one of claims 1 to 3, wherein the polyolefin-derived polymer is polyethylene (PE).

5. Use according to claim 4, wherein the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE).

6. Use according to any one of claims 1 to 5, wherein the enzyme is isolated from *Galleria mellonella,* preferably, from *G. mellonella* saliva.

7. A method for biodegrading a polyolefin-derived polymer, or a material comprising a polyolefin-derived polymer, comprising contacting:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1, or
- a host cell comprising a nucleotide sequence encoding said enzyme, or
- a composition comprising said enzyme or said host cell,
with a polyolefin-derived polymer or a material comprising a polyolefin-derived polymer.

8. Method according to claim 7, wherein the method is carried out at room temperature, preferably, at room temperature in an aqueous solution with a neutral pH.

9. Method according to claims 7 or 8, wherein the amino acid sequence comprises, or consists of, the sequence SEQ ID NO: 1.

10. Method according to any one of claims 7 to 9, wherein the composition further comprises a second enzyme which comprises an amino acid sequence having a sequence identity of, at least, 60, 65, 70, 75, 80, 85, 90, 91, 92, 93, 94, 95, 96, 97, 98 or 99% with SEQ ID NO: 2.

11. Method according to any one of claims 7 to 10, wherein the polyolefin-derived polymer is polyethylene (PE).

12. Method according to claim 11, wherein the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE).

13. Method according to any one of claims 7 to 12, wherein the enzyme is isolated from *G. mellonella,* preferably, from *G. mellonella* saliva.

14. Method for obtaining by-products derived from the biodegradation of a polyolefin-derived polymer, comprising:
(a) contacting a polyolefin-derived polymer with
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1, or
- a host cell comprising a nucleotide sequence encoding said enzyme, or
- a composition comprising said enzyme or said host cell, and
(b) isolating the by-products obtained from the culture resulting from step (a).

15. Method according to claim 14, wherein the by-products are selected from the list consisting of: butane, 2,3-Butanediol, trimethylslyl (TMS) derivative, sebacic acid, 2-ketones from 10 to 22 carbons, and a small aromatic compound recognizable as benzenepropanoic acid.
